Europäisches Patentamt

European Patent Office

Office européen des brevets

(11) Publication number: **0 397 998**
**A1**

## EUROPEAN PATENT APPLICATION

(21) Application number: 90105829.7

(22) Date of filing: 27.03.90

(51) Int. Cl.5: **A61F 13/04, A41D 31/02**

(30) Priority: 19.05.89 US 354644
22.09.89 US 411072

(43) Date of publication of application:
**22.11.90 Bulletin 90/47**

(84) Designated Contracting States:
**DE FR GB SE**

(71) Applicant: **W.L. GORE & ASSOCIATES, INC.**
**551 Paper Mill Road P.O. Box 9206**
**Newark, DE 19714(US)**

(72) Inventor: **Keller, Mary Lou**
**8 Clarison Court**
**Newark, Delaware 19714(US)**

(74) Representative: **Klunker . Schmitt-Nilson .**
**Hirsch**
**Winzererstrasse 106**
**D-8000 München 40(DE)**

(54) **Conformable protective padding.**

(57) The protective padding (10), characterized by liquid-water impermeability and water vapor-permeability is provided. The padding comprises a middle layer of stretchable padding (12) and top and bottom layers of breathable films (14). If the inner layer is secured under tension a conformable product results which is useful in some applications. The padding may be used, for example, as the underpadding for orthopedic immobilizing devices and as insulation capable of withstanding water immersion.

Fig. 1

## FIELD OF THE INVENTION

This invention relates to a conformable protective padding that provides cushioning and insulation. The padding is liquid water-impermeable, but is water vapor-permeable. The padding is particularly useful underneath orthopedic casts and braces.

## BACKGROUND OF THE INVENTION

Materials used for padding have included natural materials such as cotton, wool and down; and synthetics such as polyester fiberfill and foams. The major functions of padding are cushioning, filling, and insulation. Medical uses for paddings include applying or relieving pressure and absorption of excess moisture. Currently available paddings lose some of their functionality when immersed in water. Water can cause padding to compress or to shift due to weight gain. It also alters the insulating properties of most paddings and can cause extreme discomfort. in addition, a water saturated padding is no longer useful for transfer of fluids away from the skin. This loss of function is especially critical when the padding is to be worn by a person for extended periods. Prolonged wetness next to skin causes skin breakdown and possible infection sites. It is therefore desirable to make a padding which will not be adversely affected by exposure to water. It is also desirable to make a padding that will allow the transfer of vapors so that perspiration and other body fluids can escape.

In the past, several methods have been used to overcome some of the drawbacks of padding. Quilting has been used to prevent padding from shifting, while maintaining the high loft and bulkiness desirable in padding. Bonding padding to a fabric backing also prevents shifting. Fiberfill consists of crimped fibers to help maintain fluffiness and air space, even upon exposure to water. Hollow fibers also contain air space which add insulating value. Synthetic fibers are generally less absorbent than natural ones and have been used to wick fluids away from the skin into more absorbent backings. However, fluid saturation results in loss of function.

Another desirable attribute of padding materials is that they conform to the area they are meant to protect. A conformable product has several advantages over a more rigid product. It provides more uniform coverage over the area it is protecting; there is less gapping and, therefore, less opportunity for entry of contaminants when used for wound protection; there is decreased risk of pressure sores resulting from folds and creases; there is less restriction of movement. Other terms for conformability include extensibility, and flexibility. The ability to conform is often equated with comfort in apparel uses where it offers the advantages of fit, reduced pucker, improved wrinkle resistance, the need for fewer sizes, and greater design flexibility.

Padding is used under immobilizing orthopedic casts or braces. Traditional cast underpadding consists of cotton or polyester wraps in 2 inch to 6 inch widths. Care is taken when applying the underpadding to avoid folds or creases which can cause pressure sores to the skin. The immobilizing material applied on top of this padding is usually made of plaster of paris or polyurethane coated fiberglass. Cast wearers are usually told not to immerse the cast in water. If a traditionally padded polyurethane cast is immersed, the padding remains wet for many hours, and the result is skin maceration. In some cases, this maceration can lead to skin breakdown and infection by bacteria or fungi.

Cast material manufacturers caution against water immersion and state that if a cast is wet it should be completely dried with a hair dryer. This drying process can take several hours and patient compliance is generally low. Some cast wearers use plastic bags with rubber bands around the end to keep water away from the cast while showering. Water often enters the bag and wets the padding which causes discomfort to the wearer. As a result most cast wearers do not get their cast wet and spend the time they are in a cast without a normal shower or bath. In addition, cast wearers cannot swim or use any form of hydrotherapy.

It is widely recognized that paddings should be "breathable" to be comfortable, i.e. should allow water vapor to pass through. However, it is not necessary that air pass through the padding for it to be comfortable, only that water vapor from perspiration or other sources be transmitted from the skin outwards through the padding. The terms "breathability" and "the ability to transport interior moisture vapor to the external environment" are used interchangeably herein.

In addition, there are instances where thermal insulation in apparel or outdoor gear should be kept completely dry. To date, insulations are protected only from one side. However, when perspiration is heavy, or there is risk of immersion, a completely waterproof insulation is desirable. If this insulation can conform to the body, the advantages of a close fit can be added to the water resistant aspects of the product.

It would be desirable to provide a conformable padding for use as herein above described that is liquid water-impermeable and water vapor-permeable. To aid in conformability, it is important that the padding be stretchable.

## SUMMARY OF THE INVENTION

This invention provides a liquid water-impermeable and water vapor-permeable protective stretchable or extensible padding material that is conformable. The material comprises a middle layer of stretchable padding; and a top and bottom layer, each comprising a water-impermeable and water vapor-permeable film.

The middle layer, or each outer layer, can also be pre-tensioned before being combined.

The material, i.e. the three layered material, can also be tensioned which results in even greater subsequent extensibility.

## BRIEF DESCRIPTION OF THE DRAWINGS

Figure 1 shows a cross section of the protective padding.

Figure 2 shows a top view of a quilted protective padding.

Figure 3 shows a cross section of the padding of Figure 2 taken along lines 3-3 of Figure 2.

Figure 4 shows a cross section of the padding described in Example 1.

Figure 5 shows the protective padding of Figure 1 in place underneath a cast around a human leg.

## DESCRIPTION OF THE INVENTION

As previously described, this invention provides a liquid water-impermeable and water vapor-permeable, conformable protective stretchable padding material. The padding consists of stretchable padding materials, such as flexible open celled polyurethane foams. By the term "stretchable" is meant that the padding is capable of being stretched at least 6%, preferably 10% of its original length, and then returning to its originallength. The protective layers protect the padding material, and can be a polymeric film or laminate that encapsulates the padding. The outer protective layers provide a waterproof, "breathable" barrier, while the inner padding layer provides a cushioning and insulating flexible filler. When the inner layer is secured under tension within the outer layers the resulting product can be more conformable than if the inner layer is not pre-extended. This is particularly so when the middle padding layer is polyurethane foam. Tensioning seems to break some of the bonds of the foam and allows easier subsequent extension. The inner layer must have good recovery characteristics to provide a desirable product. Alternatively, the outer layers can be tensioned, i.e. stretched before combining with the pad, i.e. pre-stretched, or once the padding material of the invention is made, the end product can be stretched or tensioned. This tensioning appears to make the final product even more stretchable when subsequently tensioned again. This can be advantageous.

The outer layers are made of a liquid water-impermeable, water vapor-permeable material, such as microporous expanded polytetrafluoroethylene (PTFE) described in U.S. Patents 3,953,566 and 4,187,390; and expanded PTFE coated with hydrophilic impregnants and layers, such polyurethane coatings being described in U.S. Patent 4,194,041.

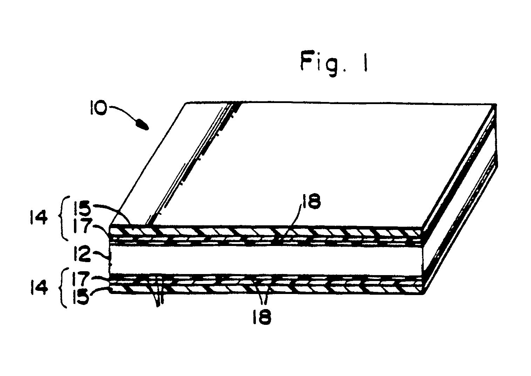

Referring to Figure 1, laminate 10 comprises middle layer of stretchable padding 12, such as a polyurethane foam or polyester or it can be a woven, knitted or non-woven material. Bonded to each side of middle layer 12 is a layer 14 containing a sheet 15 of water-impermeable, water vapor-impermeable material, such as expanded porous polytetrafluoroethylene. Layers 14 are bonded to layer 12 by means of an adhesive 18 as shown between the middle layer 12 and the top and bottom layers 14. Layers 14 as shown comprises a porous hydrophobic layer 15 and a breathable hydrophilic layer 17 as disclosed in U.S. Patent 4,194,041, and this is preferred for certain uses. However, the porous, polyolefin layers 15 may be bonded directly to the middle layer 12 by adhesive 18. Adhesive layer 18 can be a reactive polyurethane prepolymer.

In addition, it may also be desirable to divide the protective padding into smaller units or cells through quilting or embossing. This is useful not only to prevent shifting of the padding but also to protect each cell from water which may enter through a hole or defect in adjacent cells. This allows the protective padding to be cut or trimmed without violating the integrity of the whole pad.

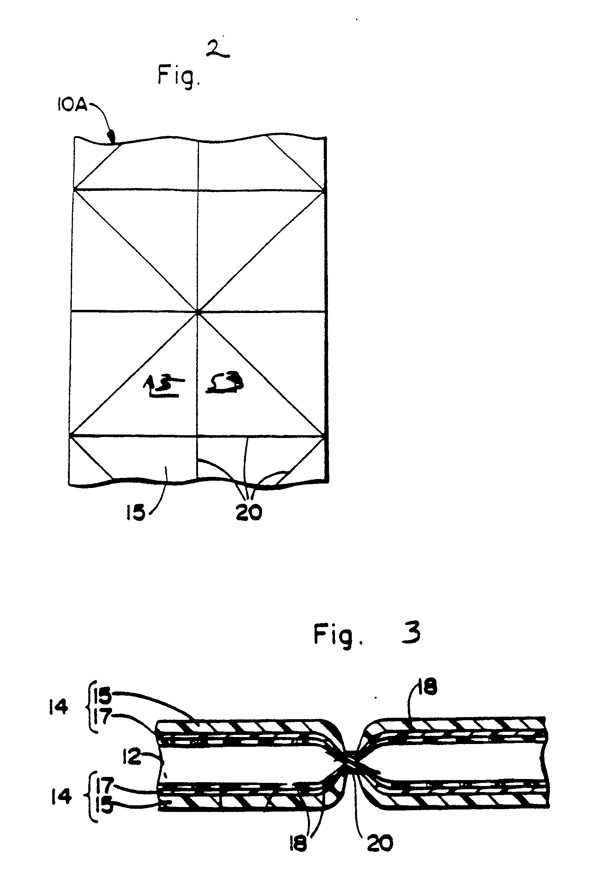

Figure 2 is a top plan view of a quilted embodiment 10 of padding for use in an orthopedic or a clothing garment, wherein the quilted pattern is in the form of triangles bounded by quilt lines 26 and Figure 3 shows a cross-sectional view taken along line 3-3 of Figure 2.

The protective padding can be manufactured by sealing the edges of the outer layers, i.e. around the periphery of the padding, to completely encompass the middle padding layer. This sealing can be done in many ways, for example, using heat, ultrasonic welding, or adhesives. Quilting can be done if thermoplastic materials are used as the padding filler and heat or ultrasound can be used to seal the edges and to create individual cells.

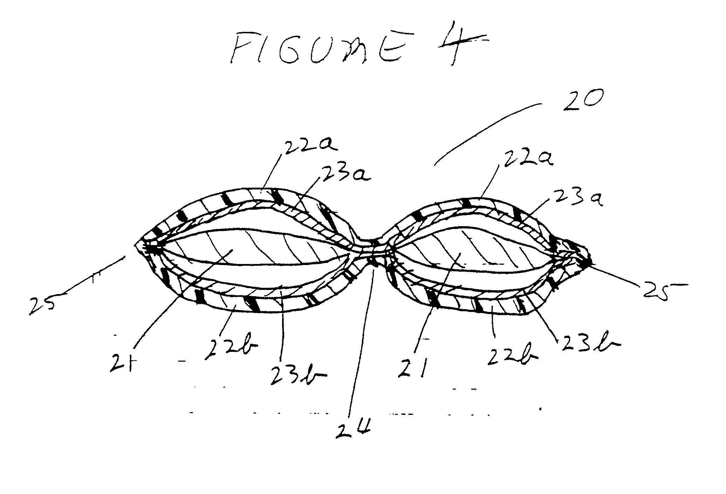

Figure 4 depicts a cross section of the embodiment of the invention described in Example 1. The protective padding comprises a middle layer of stretchable padding material 21 which can be polyurethane foam; but also could be a polyester, and can be woven, non-woven or knit form, and top outer protective layer, 22a and bottom outer protective layer 22b each made of a sheet of water-impermeable, water vapor-permeable material (which is porous, expanded polytetrafluoroethylene film) and each of which is coated with hydrophilic impregnant 23a and 23b (which is a hydrophilic breathable polyurethane). The top and bottom outer protective layers are bonded to the middle layer 21 at quilt bond point 24 by simply thermally sealing the materials together, and at outer periphery bond seams 25. The coated water-impermeable, water vapor-permeable top and bottom outer protective layers and the middle padding layer are not bonded or attached to each other at any other points. The padding can also be made by simply folding the protective material over the center padding and bonding at the seam.

The protective padding operates under several principles. Firstly, the outer layers do not allow liquid water to pass to the inner layers. Therefore, water will drain out or will pass through the film only after it has been vaporized. Additionally, the body heat of the wearer causes this evaporation process to occur and creates a one way path for water vapor. If the inner padding becomes moist the moisture is still directed outwards through the cast, not back towards the person. Therefore, padding encapsulated by the aforementioned outer layer films does not experience the same water weight gain that unprotected padding does, and the wearer does not have to wait as long to experience dryness. The skin surface dries much faster with the protected padding than with unprotected padding. Perspiration is allowed to pass through the padding without moisture remaining on the skin. Traditional padding remains damp for many hours, but the protective padding dries quickly. This provides the wearer with a great deal of comfort.

Many applications can be envisioned for such a protective padding. In addition to outstanding insulation, it provides for fast skin surface drying. Any use which requires the skin to be subjected to wet or damp conditions for prolonged periods would benefit from a padding which transfers water vapor away from the skin. Of special interest is the use of this protective padding to protect persons required to wear an orthopedic cast or brace. There are many benefits of using a waterproof, breathable cast underpadding. Such a padding allows cast or brace wearers to shower or swim while immobilized without special precautions or drying procedures. This is advantageous not only for hygenic purposes, but also for therapeutic modalities. Odor and itching is reduced for cast patients if washing is allowed on a normal schedule. Cast wearers could engage in activities which may cause perfuse perspiration without the discomfort of wet padding. Use of hydrotherapy could aid in the healing process, as it does in some other injuries which do not require immobilization.

The protective padding can be applied in a wrap formation similar to traditional cast underpaddings, or it can be configured to fit around the damaged limb in a single layer. The thinness of the film is essential to avoid bulky folds which can cause pressure sores on the skin.

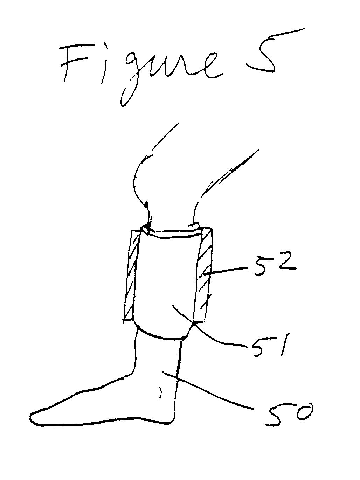

Referring to Figure 5, protective padding 51 of the invention is placed around leg 50 and orthopedic cast 52 (shown cut away) is placed around padding 51.

In addition, many athletes require the use of supportive braces to continue their training. Use of the padding of this invention prevents perspiration from being trapped next to the skin following physical exertion. Alternatively, the padding can be incorporated directly into the brace perhaps with a fabric laminated to the film.

In addition, this padding can be used in manufacturing apparel or outdoor gear such as tents, sleeping bags, etc. to provide a product which can withstand water immersion.

In all of the above applications, the attribute of conformability is desirable. For example, when used as a padding under orthopedic casts an extensibility of between 20% and 35% is desirable to conform easily around the heel or elbow of a patient. Most major manufacturers of polyurethane coated knitted fiberglass casting tapes are now commercializing cast materials with this range of extensibility. Another example is use in apparel, where a flexibility of 13%-16% is required across the shoulders if a garment is to be comfortable.

## EXAMPLES

Example 1

A stamp press set to 750 psi pressure and a temperature of 270°C was equipped with a die containing a grid pattern of 1/2 inch squares, each square being 1/2 inch deep. Two layers of porous, expanded polytetrafluoroethylene film with hydrophilic impregnants (thickness = 0.0015 inches) with a layer of polyurethane foam padding (thickness = 0.015 inches) in between them were placed under the preheated stamp press. The press was then lowered onto the three layered construction for six seconds. The resulting product was quilted, with each seam withstanding a low pressure hydrostatic test of 1 psi for 3 minutes. In the hydrostatic test, a quilted cell was punctured and the punctured side exposed to hydrostatic pressure conditions. Thus, after the stated time, the adjacent cells were opened and inspected for water infiltration.

The product was slit to 2 inch, 3 inch, and 4 inch widths, each one 4 yards long. A sample, 1 inch wide and 12 inches long, was cut for testing. One end of the sample was secured in a clamp and increasing loads were placed on the other end. The product exhibited an extensibility or stretchability of about 6% at 1 pound of load, and about 10% at 2 pounds of load. Final product thickness varied between 0.018 and 0.055 inches. The resulting padded liner was worn under a polyurethane coated fiberglass cast. The wearer showered and swam on several occasions, and felt dry approximately one hour after wetting. No skin maceration was noted after several days of repeated immersions. No water leakage had occurred into the padding.

Example 2

A stamp press set of 750 psi pressure and a temperature of 270° C was equipped with a special die containing a grid pattern of 1/2 inch squares, each square being 1/2 inch deep. Two layers of porous, expanded polytetrafluoroethylene film with hydrophilic impregnants with a layer of polyurethane foam padding in between them were placed under the preheated stamp press. The inner layer of polyurethane foam was stretched to near breakage, approximately 150% elongation, but the outer layers were not stretched. The press was then lowered onto the three layered construction for six seconds. The resulting product was quilted, with each seam withstanding a low pressure hydrostatic test of 1 psi for 3 minutes. The product exhibited an extensibility or stretchability of about 26% at 1 pound of load and about 45% at 2 pounds of load.

Example 3

A stamp press set of 750 psi pressure and a temperature of 270° C was equipped with a special die containing a grid pattern of 1/2 inch squares, each square being 1/2 inch deep. Two layers of porous expanded polytetrafluoroethylene film with hydrophilic impregnants with a layer of polyurethane foam padding in between them were placed under the preheated stamp press. The press was then lowered onto the three layered construction for six seconds. The resulting product was quilted, with each seam withstanding a low pressure hydrostatic test of 1 psi of 3 minutes. The product was then stretched under approximately 10 pounds of load. The product exhibited an extensibility or stretchability of about 8% at 1 pound of load and about 18% at 2 pounds of load.

Example 4

A stamp press set to 750 psi pressure and a temperature of 270° C was equipped with a special die containing a grid pattern of 1/2 inch squares, each square being 1/2 inch deep. Two layers of porous expanded polytetrafluoroethylene film with hydrophilic impregnants with a layer of polyurethane foam padding in between them were placed under the preheated stamp press. The PTFE outer layers had been pre-stretched to 25% beyond normal. The press was then lowered onto the three layered construction for three seconds. The resulting product was quilted, with each seam withstanding a low pressure hydrostatic test of 1 psi for 3 minutes. The product exhibited an extensibility or stretchability of about 14% at 1 pound of load and about 23% at 2 pounds of load.

Example 5

A stamp press set to 800 psi pressure and a temperature of 200° C was equipped with a special die containing a grid pattern of 1/2 inch squares, each square being 1/2 inch deep. Two layers of "Hyperm", polyurethane film (thickness = 0.0008 inches) with a layer of polyurethane foam padding in between them were placed under the preheated stamp press. The press was then lowered onto the three layered construction for ten seconds. The product exhibited an extensibility or stretchability of about 6% at 1 pound of load and about 9% at 1.5 pounds of load.

Example 6

A stamp press set to 750 psi pressure and a temperature of 250° C was equipped with a die containing a grid pattern of 1/2 inch squares, each square being 1/2 inch deep. Two layers of porous exanded polytetrafluoroethylene film with hydrophilic impregnants (thickness = 0.0015 inches) with a layer of Lycra® spandex fabric (thickness = 0.016 inches) in between them were placed under the preheated stamp press. The inner layer of spandex was stretched to approximately 150% elongation, but the outer layers were not stretched. The press was then lowered onto the three layered construction for three seconds. The product exhibited an extensibility or stretchability of about 16% at 1 pound of load, and about 19% at 1.5 pounds of load.

## Claims

1. A protective padding, characterized by liquid-water impermeability and water vapor-permeability comprising:

   (a) a middle layer of stretchable padding, and

   (b) a top and bottom layer each comprising a water-impermeable and moisture-vapor-permeable film.

2. A protective padding as defined in claim 1, where the padding is in a tensioned condition.

3. A protective padding of claims 1 or 2, wherein the middle layer is a flexible, open-celled polyurethane foam.

4. A protective padding as defined in at least one of claims 1 to 3, where the top and bottom layers are sealed at the edges to encompass the middle layer.

5. A protective padding as defined in claims 1 or 2, where the middle layer is a thermoplastic padding which is secured within the outer layers.

6. A protective padding as defined in claims 4 or 5, where an additional immobilizing layer is applied on top of the protective padding.

7. A protective padding as defined in claim 6, where the additional immobilizing layer is a water compatible orthopedic cast material.

8. A protective padding as defined in at least one of the preceeding claims, where the additional immobilizing layer is an orthopedic brace.

9. A protective padding as defined in at least one of the preceeding claims, where the padding is incorporated into a garment or outdoor gear.

10. A protective padding of claim 1 wherein the top and bottom layers are joined together in discrete quilt patterns by heat fusing the middle layer only along the peripheral boundaries of the quilt pattern compressing the outer layers together.

Fig. 1

Fig. 2

10A

15

20

Fig. 3

14 { 15
17

18

12

14 { 17
15

18    20

FIGURE 4

Figure 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 296 364 (L.J. RAUTENBERG et al.) <br> * Page 3, lines 25-29; page 3, line 38 - page 4, line 3; page 4, lines 45-49; page 8, lines 21-35; figure 2A. * | 1-5,9 | A 61 F 13/04 <br> A 41 D 31/02 |
| Y | EP-A-0 289 681 (W.L. GORE & ASSOCIATES) <br> * Column 2, lines 2-37; claim 4 * | 6-7 | |
| Y | US-A-4 788 972 (A.O.V. DEBUSK) <br> * Column 1, line 62 - column 2, line 12; figure 3 * | 8 | |
| Y | FR-A-2 080 019 (FABRIQUE DE SOIERIES ANDRE & SERGE FERRARI) <br> * Page 1, lines 1-4,20-26; page 2, line 40 - page 3, line 6; page 3, lines 14-18; figures 1,3 * | 10 | |
| A | | 4-5,9 | |
| A | FR-A-1 309 622 (MICHEL ET PIERRE LAPOUTRE) <br> * Summary * | 1-3,5,9 | TECHNICAL FIELDS SEARCHED (Int. Cl.5) <br><br> A 61 F <br> A 41 D |

The present search report has been drawn up for all claims

| Place of search | Date of completion of the search | Examiner |
|---|---|---|
| THE HAGUE | 03-08-1990 | NICE P.R. |